# EUROPEAN PATENT APPLICATION

(11) **EP 4 349 860 A1**
(43) Date of publication of application: **10.04.2024**
(21) Application number: 22382940.9
(22) Date of filing: 06.10.2022
(51) Int. Cl.: C07K 16/10, A61P 31/14

(54) **ANTI-SARS-COV-2 ANTIBODIES**

(71) Applicant: Fundació Institut Hospital Del Mar D'Investigacions Mèdiques (IMIM), 08003 Barcelona (ES); Fundació Privada Institut de Recerca de la SIDA-Caixa, 08916 Badalona (ES); Fundació Centre de Regulació Genòmica, 08003 Barcelona (ES); Fundació Institut d'Investigació en Ciències de la Salut Germans Trias i Pujol (IGTP), 08916 Badalona (ES)
(72) Inventor: MAGRI, Giuliana, 08004 BARCELONA (ES); TEJEDOR VAQUERO, Sonia, 33010 OVIEDO (ES); DE CAMPOS-MATA, Leire, 01191 ÁLAVA (ES); CAROLIS, Carlo, 08348 CABRILS (ES); BLANCO ARBUÉS, Julià, 08916 BADALONA (ES); TRINITÉ, Benjamin, 08916 BADALONA (ES); PRADENAS SAAVEDRA, Edwards, 08916 BADALONA (ES); MODREGO GUILLEN, Andrea, 28049 MADRID (ES); SANTIAGO HERNANDEZ, Cesar Augusto, 28049 MADRID (ES); ARRANZ AVILA, Rocio, 28049 MADRID (ES)
(74) Representative: ZBM Patents - Zea, Barlocci & Markvardsen

(57) **Abstract**

The present invention refers to antibodies or antigen-binding fragments thereof that bind the SARS-CoV-2 spike protein with high affinity and subsequent high neutralizing activity against SARS-CoV-2. It also discloses polynucleotides and expression vectors encoding said antibodies or antigen-binding fragments, and host cells comprising said nucleotides and vectors, as well as their use in treatment or prevention of a disease caused by a SARS-CoV-2.

## Description

### Technical Field

The present invention relates to antibodies and antigen-binding fragments that bind specifically to coronavirus spike proteins and methods for treating or preventing coronavirus infections with said antibodies and fragments, as well as to methods for the diagnosis of infections caused by this virus.

### Background Art

The severe acute respiratory syndrome coronavirus 2 (SARS-CoV-2) infection and coronavirus disease 2019 (COVID-19) reached pandemic level in 2020. Due to the mutation index of the virus, multiple variants have emerged from the first declared case in 2019. Some of these variants present mutations in their spike protein receptor binding domain (RBD) which substantially increase the binding affinity of its complex with the cellular receptor ACE2 (this union allows the entrance of the virus into the target human cell), thus contributing to rapid spread in human populations. As a result, several thousands of million people have been infected and some million have died worldwide.

Since the reach of pandemic level in March 2020, scientists, health authorities, pharmaceutical companies and the governments have accelerated not only the study of SARS-CoV-2 and its variants, but also the generation of fast diagnostic and prognostic tests and assays, and therapeutic approaches to stop the devastating effects of the infection and, consequently, of the pandemic.

As a result, different vaccines have been developed and marketed against COVID-19, as well as compounds that block the infective and replicative cycle of the virus. An example is the antibodies disclosed in patent document US10787501B1 (Regeneron Pharmaceuticals, Inc), which proved to be neutralizing against the virus. Also, widely known for treating COVID-19 is the use of the approved actives sarilumab, tocilizumab, gimsilumab, tixagevimab, cilgavimab, PF-07321332, ritonavir, regdanvimab, casirivimab, imdevimab, remdesivir, and sotrovimab, among other strategies such as antibodies or antigen-binding fragments that bind the serine protease TMPRSS2, which is also implicated in the virus entry into the target cells.

Nevertheless, severe cases continue to pour into hospitals owing to a combination of the limited efficacy of vaccines, the large proportion of unvaccinated population, the individual susceptibility and comorbidity, and the appearance of new variants of the virus. Importantly, apart from the concerning mutations in these variants which increased their transmissibility and/or virulence, the variants can also accumulate escape mutations, which make that previously infected or vaccinated individuals may be also affected as their immune system is not able to rapidly recognize and eliminate the pathogen, such as a virus, as it would do with the variant or variants with which has been already in contact, i.e. immunized.

Additionally, already developed vaccines, which are actually designed against SARS-CoV-2 original strain, and treatments have shown to lose efficacy against variants due to similar reasons. Mutations occurred in Omicron variants BA.1, BA.2, BA.4, and BA.5 strongly affected the efficacy of current treatments. Some antibodies which proved to efficiently neutralize all previous different variants, showed an important decrease in the neutralization activity against the Omicron variants, as seen for example in Tongqing Zhou et al., "Structural basis for potent antibody neutralization of SARS-CoV-2 variants including B.1.1.529", Science 2022, vol. 376, issue 6591, https://doi.org/10.1126/science.abn8897, and in Bruel, T. et al. "Serum neutralization of SARS-CoV-2 Omicron sublineages BA.1, BA.2, BA.4 and BA.5 in patients receiving monoclonal antibodies". Nat Med 2022, https://doi.org/10.1038/s41591-022-01792-5.

Thus, there is still an urgent need of developing new therapeutic approaches that prove to be useful independently of the SARS-CoV-2 variant. In addition, there is a need of powerful diagnostic tools able to detect low amounts of the variants.

### Summary of Invention

During the investigations that led to the present invention, the inventors developed an antibody with outstanding and similar neutralizing activity against all SARS-CoV-2 variants, including omicron variants (BA.1, BA.2, BA.4, BA.5). This is of utmost importance, as it would not only allow treating patients infected with current variants, but also defining homogeneous doses of the same antibody and independently of the patient's SARS-CoV-2 variant.

Thus, a first aspect of the invention is an antibody comprising a light chain variable region (LCVR) that comprises three light chain complementarity determining regions (LCDRs) set forth in SEQ ID NO: 1 (LCDR1), SEQ ID NO: 2 (LCDR2), and SEQ ID NO: 3 (LCDR3); and a heavy chain variable region (HCVR) that comprises three heavy chain complementarity determining regions (HCDRs) set forth in SEQ ID NO: 4 (HCDR1), SEQ ID NO: 5 (HCDR2), and SEQ ID NO: 6 (HCDR3). This antibody binds the receptor binding domain of the SARS-CoV-2 spike protein with high affinity, for example, a binding affinity expressed as K_{D} (Dissociation Constant), of less than about 10⁻⁹ M, or of less than about 10⁻¹⁰ M, or of less than about 10⁻¹¹ M, as measured by real-time surface plasmon resonance, e.g. BIACORE^{™}. K_{D} refers to the dissociation constant for an antibody antigen interaction.

The antibody also presents high neutralization activity, for example, with half maximal inhibitory concentration (IC₅₀) values below 200 ng/ml, or below 100 ng/ml, or below 50 ng/ml, or below 40 ng/ml, or below 20 ng/ml, or below 12 ng/ml as measured in pseudotyped virus neutralization assays.

Also contemplated are fragments comprising a light chain variable region (LCVR) that comprises three light chain complementarity determining regions (LCDRs) set forth in SEQ ID NO: 1 (LCDR1), SEQ ID NO: 2 (LCDR2), and SEQ ID NO: 3 (LCDR3); and a heavy chain variable region (HCVR) that comprises three heavy chain complementarity determining regions (HCDRs) set forth in SEQ ID NO: 4 (HCDR1), SEQ ID NO: 5 (HCDR2), and SEQ ID NO: 6 (HCDR3).

The second aspect of the invention is a polynucleotide encoding the antibody or antigen-binding fragment thereof as defined in the first aspect.

The third aspect of the invention provides an expression vector comprising the polynucleotide as defined in the second aspect.

A host cell can be transformed or transfected with the polynucleotide(s) and/or the vector(s) of the invention. Thus, a fourth aspect of the invention is a host cell comprising the polynucleotide or the vector as defined in the second and third aspect.

The fifth aspect refers to a method for preparing the antibody or antigen-binding fragment thereof as defined in the first aspect.

The sixth aspect relates to a pharmaceutical composition comprising a therapeutically effective amount of the antibody, or antigen binding fragment thereof as defined in the first aspect, or a nucleotide as defined in the second aspect, or an expression vector as defined in the third aspect, and a pharmaceutically acceptable excipient and/or carrier.

The seventh aspect of the invention relates to an antibody or antigen binding fragment thereof as defined in the first aspect, or a nucleotide as defined in the second aspect, or an expression vector as defined in the third aspect, or the pharmaceutical composition as defined in the sixth aspect, for use in therapy. In other words, they are for use as a medicament.

The seventh aspect can also be formulated as the use of the antibody or antigen binding fragment thereof as defined in the first aspect, or a nucleotide as defined in the second aspect, or an expression vector as defined in the third aspect, or the pharmaceutical composition as defined in the sixth aspect, for the manufacture of a medicament. This aspect can also be formulated as a method for the prevention and/or treatment of a disease, the method comprising administering a therapeutically effective amount of the antibody or antigen binding fragment thereof as defined in the third aspect, or a nucleotide as defined in the second aspect, or an expression vector as defined in the third aspect, or the pharmaceutical composition as defined in the sixth aspect, in a subject in need thereof.

The eighth aspect of the invention relates to the use of the antibody or antigen binding fragment as defined in the first aspect for the detection of SARS-CoV-2 in an isolated biological sample from a subject.

### Detailed description of the invention

Unless defined otherwise, all technical and scientific terms used herein have the same meaning as is commonly understood by one of skill in the art to which this invention belongs at the time of filling. However, in the event of any latent ambiguity, definitions provided herein take precedent over any dictionary or extrinsic definition. Further, unless otherwise required by context, singular terms shall include pluralities and plural terms shall include the singular.

As used herein, the indefinite articles "a" and "an" are synonymous with "at least one" or "one or more." Unless indicated otherwise, definite articles used herein, such as "the" also include the plural of the noun.

The term "SARS-CoV-2" refers to the SARS-CoV-2 virus of the coronavirus family, the emerged coronavirus which was identified as the cause of a serious outbreak starting in Wuhan, China, and which rapidly spread to other areas of the globe. SARS-CoV-2 has also been known as 2019-nCoV and Wuhan coronavirus. It binds via the viral spike protein, in particular to the receptor binding domain (RBD) of the spike protein, to the human host cell receptor angiotensin-converting enzyme 2 (ACE2). The spike protein also binds to and is cleaved by the serine protease TMPRSS2, which activates the spike protein for membrane fusion of the virus. Several variants of the SARS-CoV-2 have emerged from the ancestral WH1 (or Wuhan) variant. The ancestral WH1 variant is also referred herein as wild type (WT) or original variant.

The terms "variants" refers to the SARS-CoV-2 variants that have emerged from the ancestral WH1 or Wuhan variant, including but not limited to D614G, Alpha, Beta (β), Iota, Lambda, Gamma (γ), Delta (δ), Mu, and Omicron. The term "Omicron" includes but is not limited to Omicron variants or sublineages BA.1, BA.2, BA.4 and BA.5. There are other nomenclatures used to refer to the different SARS-CoV-2 variants. For example, the following variants can be also cited with the following codes: Alpha - B.1.1.7; Beta - B.1.351.1; Gamma - P.1; Delta - B.1.617.2; Mu - B.1.621; Omicron - B.1.1.529 (BA.1: B.1.1.529.1,BA.2: B.1.1.529.2, BA.4: B.1.1.529.4 and BA.5: B.1.1.529.5); Lambda - C.37; and Iota - B.1.525. Further information can be found in the following webpage: https://cov-lineages.org/lineage list.html (accessed on August 26th, 2022 - 11:38 am CET). The term "variant" also includes variants of concern (VOC)s, which are variants for which there is evidence of an increase in transmissibility, more severe disease (for example, increased hospitalizations or deaths), significant reduction in neutralization by antibodies generated during previous infection or vaccination, reduced effectiveness of treatments or vaccines, or diagnostic detection failures. Also, VOCs category is used for variants of the virus where mutations in their spike protein receptor binding domain (RBD) substantially increase the binding affinity of its complex with the cellular receptor ACE2, thus contributing to rapid spread in human populations. For example, particular VOCs are BA.4 and BA.5 Omicron variants.

The term "SARS-CoV-2-S", also called "S" or "S protein" refers to the spike protein of SARS-CoV-2 coronavirus, and refers to the specific S proteins of SARS-CoV-2-S. The SARS-CoV-2-Spike protein is a 1273 amino acid type I membrane glycoprotein which assembles into trimers that constitute the spikes or peplomers on the surface of the enveloped coronavirus particle. The protein has two essential functions, host receptor binding and membrane fusion, which are attributed to the N-terminal (S1) and C-terminal (S2) halves of the S protein. SARS-CoV-2-S binds to its cognate receptor via a receptor binding domain (RBD) present in the S1 subunit. The amino acid sequence of full-length SARS-CoV-2 spike protein of the WH1 variant is exemplified by the amino acid sequence provided in SEQ ID NO: 13. The term "SARS-CoV-2-S" includes protein variants of SARS-CoV-2 spike protein isolated from different SARS-CoV-2 variants as well as recombinant SARS-CoV-2 spike protein or a fragment thereof. SEQ ID NO: 13 is

The term "SARS-CoV-2 infection" as used herein, refers to infection with SARS-CoV-2. The term includes SARS-CoV-2 respiratory tract infections, often in the lower respiratory tract. Symptoms can include high fever, dry cough, shortness of breath, pneumonia, gastro-intestinal symptoms such as diarrhea, organ failure (kidney failure and renal dysfunction), septic shock, and death in severe cases. The term "COVID-19" refers to the disease caused by a SARS-CoV-2 infection.

The term "antibody", as used herein, refers to immunoglobulin molecules comprising four polypeptide chains, two heavy chains (HCs) and two light chains (LCs) inter-connected by disulfide bonds (i.e., "full antibody molecules"), as well as multimers thereof (e.g. IgM). Each heavy chain comprises a heavy chain variable region ("HCVR" or "VH") and a heavy chain constant region (comprised of domains CH 1, CH 2, and CH 3). Each light chain is comprised of a light chain variable region ("LCVR" or "VL") and a light chain constant region (CL). The VH and VL regions can be further subdivided into regions of hypervariability, termed complementarity determining regions (CDR), interspersed with regions that are more conserved, termed framework regions (FR). Each VH and VL comprises three CDRs and four FRs, arranged from amino-terminus to carboxy-terminus in the following order: FR1, CDR1, FR2, CDR2, FR3, CDR3, FR4. Heavy chain CDRs can also be referred to as HCDRs or CDR-Hs, and numbered as described above (e.g., HCDR1, HCDR2, and HCDR3 or CDR-H1, CDR-H2, and CDR-H3). Likewise, light chain CDRs can be referred to as LCDRs or CDR-Ls, and numbered LCDR1, LCDR2, and LCDR3, or CDR-L1, CDR-L2, and CDR-L3.

In general, from N-terminal to C-terminal, both light and heavy chains variable domains comprise FR1, CDR1, FR2, CDR2, FR3, CDR3 and FR4.

The terms "antigen-binding fragment" of an antibody, and the like, as used herein, include any naturally occurring, enzymatically obtainable, synthetic, or genetically engineered polypeptide or glycoprotein that specifically binds an antigen to form a complex. Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies, small modular immunopharmaceuticals (SMIPs), and shark variable IgNAR domains, are also encompassed within the expression "antigen-binding fragment," as used herein.

"Isolated" antibodies or antigen-binding fragments thereof, polypeptides, polynucleotides and vectors, are at least partially free of other biological molecules from the cells or cell culture from which they are produced. Such biological molecules include nucleic acids, proteins, other antibodies or antigen-binding fragments, lipids, carbohydrates, or other material such as cellular debris and growth medium. An isolated antibody or antigen-binding fragment may further be at least partially free of expression system components such as biological molecules from a host cell or of the growth medium thereof. Generally, the term "isolated" is not intended to refer to a complete absence of such biological molecules or to an absence of water, buffers, or salts or to components of a pharmaceutical formulation that includes the antibodies or fragments.

In the present invention the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

As an illustration, by a polypeptide having an amino acid sequence having at least, for example, 95% identity to a reference amino acid sequence of SEQ ID NO: X is intended that the amino acid sequence of the polypeptide is identical to the reference sequence except that the polypeptide sequence may include up to 5 amino acid alterations per each 100 amino acids of the reference amino acid of SEQ ID NO: X. In other words, to obtain a polypeptide having an amino acid sequence of at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

A number of mathematical algorithms for rapidly obtaining the optimal alignment and calculating identity between two or more sequences are known and incorporated into a number of available software programs. For purposes of the present invention, the sequence identity between two amino acid sequences is preferably determined using algorithms based on global alignment, such as the Needleman-Wunsch algorithm (Needleman and Wunsch, J. Mol. Biol. 48: 443-453, 1970. DOI: 10.1016/0022-2836(70)90057-4), preferably implemented in the Needle program of the EMBOSS package (EMBOSS: The European Molecular Biology Open Software Suite, Rice et al., , Trends Genet. 16: 276-277, 2000. DOI: 10.1016/s0168-9525(00)02024-2); or the BLAST Global Alignment tool (Altschul et al., "Basic local alignment search tool", 1990, J. Mol. Biol, v. 215, pages 403-410, 1990. https://doi.org/10.1016/S0022-2836(05)80360-2), using default settings. Local alignment also can be used when the sequences being compared are substantially the same length.

The present disclosure additionally contemplates antibodies or fragments thereof comprising conservative mutations with respect to the antibodies or fragments thereof described herein. By "conservative mutations" it is understood to mean amino acid substitutions that do not substantially affect or decrease a function of a protein. Such as the ability of the protein to interact with a target protein. For examples, a SARS-CoV-2 specific antibody can include up to 1, 2, 3, 4, 5, 6, 7, 8, 9 or up to 10 conservative substitutions compared to a reference antibody sequence and retain specific binding activity and/or neutralization activity

Accordingly, a first aspect of the invention is an antibody or antigen-binding fragment thereof comprising a light chain variable region (LCVR) that comprises three light chain complementarity determining regions (LCDRs) set forth in SEQ ID NO: 1 (LCDR1), SEQ ID NO: 2 (LCDR2), and SEQ ID NO: 3 (LCDR3); and a heavy chain variable region (HCVR) that comprises three heavy chain complementarity determining regions (HCDRs) set forth in SEQ ID NO: 4 (HCDR1), SEQ ID NO: 5 (HCDR2), and SEQ ID NO: 6 (HCDR3).

In one embodiment of the first aspect, the LCVR comprises or consists of a sequence at least 80%, at least 85%, or at least 90% identical to SEQ ID NO: 7; particularly provided that the LCDRs are of sequence as set forth in SEQ ID NO: 1-3. In a particular embodiment, the LCVR comprises or consists of a sequence at least 95%, at least 96%, at least 97%, at least 98%, or at least 99% identical to SEQ ID NO: 7; particularly provided that the LCDRs are of sequence as set forth in SEQ ID NO: 1-3. In a more particular embodiment of the first aspect, the LCVR comprises or consists of the amino acid sequence set forth in SEQ ID NO: 7.

In one embodiment of the first aspect, the HCVR comprises or consists of a sequence at least 80%, at least 85%, or at least 90% identical to SEQ ID NO: 8; particularly provided that the HCDRs are of sequence as set forth in SEQ ID NO: 4-6. In a particular embodiment, the HCVR comprises or consists of a sequence at least 95%, at least 96, at least 97%, at least 98%, or at least 99% identical to SEQ ID NO: 8; particularly provided that the HCDRs are of sequence as set forth in SEQ ID NO: 4-6. In a more particular embodiment of the first aspect, the HCVR comprises or consists of the amino acid sequence set forth in SEQ ID NO: 8.

In an even more particular embodiment of the first aspect, the antibody or antigen-binding fragment thereof comprises:
- a heavy chain comprising a sequence at least 80%, at least 85%, or at least 90% identical to SEQ ID NO: 8; particularly provided that the HCDRs are of sequence as set forth in SEQ ID NO: 4-6; and
- a light chain comprising a sequence at least 80%, at least 85%, or at least 90% identical to SEQ ID NO: 7; particularly provided that the LCDRs are of sequence as set forth in SEQ ID NO: 1-3.

In an even more particular embodiment of the first aspect, the antibody or antigen-binding fragment thereof comprises:
- a heavy chain comprising a sequence at least 95%, at least 96%, at least 97%, at least 98%, or at least 99% identical to SEQ ID NO: 8; particularly provided that the HCDRs are of sequence as set forth in SEQ ID NO: 4-6; and
- a light chain comprising a sequence at least 95%, or at least 96%, at least 97%, at least 98%, or at least 99% identical to SEQ ID NO: 7; particularly provided that the LCDRs are of sequence as set forth in SEQ ID NO: 1-3.

In an even more particular embodiment of the first aspect, the antibody or antigen-binding fragment thereof comprises:
- a heavy chain comprising a sequence as set forth in SEQ ID NO: 8; and
- a light chain comprising a sequence as set forth in SEQ ID NO: 7.

In a particular embodiment, the antibody or antigen-binding fragment thereof comprises a VH1-58 and/or a VK3-20 variable domain sequences.

In an embodiment of the invention, the antibody or antigen-binding fragment thereof comprises a heavy chain constant domain. In a particular embodiment it comprises an immunoglobulin constant region. In a more particular embodiment the immunoglobulin constant region is of the type of IgA (e.g., IgA1 or IgA2), IgD, IgE, IgG (e.g., IgG1, IgG2, IgG3 and IgG4) or IgM. In a more particular embodiment it is an IgG1 constant region. In a more particular embodiment it is a human IgG1 constant region.

In an embodiment of the invention, the antibody or antigen-binding fragment thereof comprises a light chain constant domain. In a particular embodiment it comprises an immunoglobulin constant region. In a more particular embodiment, the immunoglobulin constant region is of the type of kappa (IgK) or lambda (IgL). In a more particular embodiment, it is a IgK constant region. In a more particular embodiment, it is a human IgK constant region.

In a particular embodiment, the constant region of the heavy chain comprises or consists of a sequence at least 70%, at least 75%, at least 80, or at least 85% identical to SEQ ID NO: 9. In a particular embodiment, the constant region of the light chain comprises at least 90%, at least 91%, at least 92%, at least 93%, or at least 94% identical to SEQ ID NO: 9. In a particular embodiment, the constant region of the heavy chain comprises or consists of a sequence at least 95%, at least 96%, at least 97%, at least 98%, or at least 99% identical to SEQ ID NO: 9. In a more particular embodiment, the constant region of the heavy chain comprises or consists of a sequence as set forth in SEQ ID NO: 9.

In a particular embodiment, the constant region of the light chain comprises or consists of a sequence at least 70%, at least 75%, at least 80%, or at least 85% identical to SEQ ID NO: 10. In a particular embodiment, the constant region of the light chain comprises at least 90%, at least 91%, at least 92%, at least 93%, or at least 94% identical to SEQ ID NO: 10. In a particular embodiment, the constant region of the light chain comprises or consists of a sequence at least 95%, at least 96%, at least 97%, at least 98%, or at least 99% identical to SEQ ID NO: 10. In a more particular embodiment, the constant region of the light chain comprises or consists of a sequence as set forth in SEQ ID NO: 10.

In an even more particular embodiment of the first aspect, the antibody or antigen-binding fragment thereof comprises:
- a heavy chain comprising a sequence at least 70%, at least 75%, or at least 85% identical to SEQ ID NO: 9; and
- a light chain comprising a sequence at least 70%, at least 75%, or at least 85% identical to SEQ ID NO: 10.

In an even more particular embodiment of the first aspect, the antibody or antigen-binding fragment thereof comprises:
- a heavy chain comprising a sequence at least 90%, at least 91%, at least 92%, at least 93%, or at least 94% identical to SEQ ID NO: 9; and
- a light chain comprising a sequence at least 90%, at least 91%, at least 92%, at least 93%, or at least 94% identical to SEQ ID NO: 10.

In an even more particular embodiment of the first aspect, the antibody or antigen-binding fragment thereof comprises:
- a heavy chain comprising a sequence at least 95%, at least 96%, at least 97%, at least 98%, or at least 99% identical to SEQ ID NO: 9; and
- a light chain comprising a sequence at least 95%, at least 96%, at least 97%, at least 98%, or at least 99% identical to SEQ ID NO: 10.

In an even more particular embodiment of the first aspect, the antibody or antigen-binding fragment thereof comprises:
- a heavy chain comprising a sequence as set forth in SEQ ID NO: 9; and
- a light chain comprising a sequence as set forth in SEQ ID NO: 10.

In an even more particular embodiment of the first aspect, the antibody or antigen-binding fragment comprises:
- a heavy chain comprising or consisting of a sequence at least 70%, at least 75%, at least 80%, or at least 85% identical to SEQ ID NO: 11; particularly provided that the
   HCDRs are of sequence as set forth in SEQ ID NO: 4-6; and
- a light chain comprising or consisting of a sequence at least 70%, at least 75%, at least 80%, or at least 85% identical to SEQ ID NO: 12; particularly provided that the LCDRs are of sequence as set forth in SEQ ID NO: 1-3.

In an even more particular embodiment of the first aspect, the antibody or antigen-binding fragment comprises:
- a heavy chain comprising or consisting of a sequence at least 90%, at least 91%, at least 92%, at least 93%, or at least 94% identical to SEQ ID NO: 11; particularly provided that the HCDRs are of sequence as set forth in SEQ ID NO: 4-6; and
- a light chain comprising or consisting of a sequence at least 90%, at least 91%, at least 92%, at least 93%, or at least 94% identical to SEQ ID NO: 12; particularly provided that the LCDRs are of sequence as set forth in SEQ ID NO: 1-3.

In an even more particular embodiment of the first aspect, the antibody or antigen-binding fragment comprises:
- a heavy chain comprising or consisting of a sequence at least 95%, at least 96%, at least 97%, at least 98%, or at least 99% identical to SEQ ID NO: 11; particularly provided that the HCDRs are of sequence as set forth in SEQ ID NO: 4-6; and
- a light chain comprising or consisting of a sequence at least 95%, at least 96%, at least 97%, at least 98%, or at least 99% identical to SEQ ID NO: 12; particularly provided that the LCDRs are of sequence as set forth in SEQ ID NO: 1-3.

In an even more particular embodiment of the first aspect, the antibody comprises:
- a heavy chain comprising or consisting of a sequence SEQ ID NO: 11; and
- a light chain comprising or consisting of a sequence SEQ ID NO: 12.

The amino acid reference sequences of the antibody or antigen binding fragment thereof according to the first aspect are listed in the following Table A.

**Table A**

| SEQ ID. | SEQ Name | Amino acid sequence |
|---|---|---|
| SEQ ID NO: 1 | LCDR1 | QSISSNY |
| SEQ ID NO: 2 | LCDR2 | GAS |
| SEQ ID NO: 3 | LCDR3 | QHYGGLSRWT |
| SEQ ID NO: 4 | HCDR1 | VFTFSISA |
| SEQ ID NO: 5 | HCDR2 | IWGSGNT |
| SEQ ID NO: 6 | HCDR3 | AAPYCNRTTCYDGFDL |
| SEQ ID NO: 7 | LCVR | EIVLTQSPGTLSLSPGERATLSCRASQSISSNYLAWYQQKPG QAPRLLIYGASSRATGIPDRFSGSGSGTDFTLSINRLEPEDFA MYYCQHYGGLSRWTFGQGTKVEIK |
| SEQ ID NO: 8 | HCVR | QVQLVQSGPEVKKPGTSVKVSCKAPVFTFSISAVQWVRQAR GQRLEWIGWIVVGSGNTNYAQNFQERVTITTDMSTGTVYME LSGLRSEDTAMYYCAAPYCNRTTCYDGFDLWGQGTMVTVS S |
| SEQ ID NO: 9 | Constant region Heavy Chain | ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKRVEPKSCDKTHTCPPCPAPELLGGPSVF LFPPKPKDTLMISRTPEVTCVVVDVSHEDPEVKFNWYVDGV EVHNAKTKPREEQYNSTYRVVSVLTVLHQDWLNGKEYKCKV SNKALPAPIEKTISKAKGQPREPQVYTLPPSREEMTKNQVSL TCLVKGFYPSDIAVEWESNGQPENNYKTTPPVLDSDGSFFL YSKLTVDKSRWQQGNVFSCSVMHEALHNHYTQKSLSLSPG K |
| SEQ ID NO: 10 | Constant region Light Chain | |
| SEQ ID NO: 11 | Heavy chain | QVQLVQSGPEVKKPGTSVKVSCKAPVFTFSISAVQWVRQAR GQRLEWIGWIVVGSGNTNYAQNFQERVTITTDMSTGTVYME |
| | | LSGLRSEDTAMYYCAAPYCNRTTCYDGFDLWGQGTMVTVS SASTKGPSVFPLAPSSKSTSGGTAALGCLVKDYFPEPVTVS WNSGALTSGVHTFPAVLQSSGLYSLSSVVTVPSSSLGTQTYI CNVNHKPSNTKVDKRVEPKSCDKTHTCPPCPAPELLGGPSV FLFPPKPKDTLMISRTPEVTCVVVDVSHEDPEVKFNWYVDG VEVHNAKTKPREEQYNSTYRVVSVLTVLHQDWLNGKEYKCK VSNKALPAPIEKTISKAKGQPREPQVYTLPPSREEMTKNQVS LTCLVKGFYPSDIAVEWESNGQPENNYKTTPPVLDSDGSFFL YSKLTVDKSRWQQGNVFSCSVMHEALHNHYTQKSLSLSPG K |
| SEQ ID NO: 12 | Light Chain | EIVLTQSPGTLSLSPGERATLSCRASQSISSNYLAWYQQKPG QAPRLLIYGASSRATGIPDRFSGSGSGTDFTLSINRLEPEDFA MYYCQHYGGLSRWTFGQGTKVEIKRTVAAPSVFIFPPSDEQ LKSGTASVVCLLNNFYPREAKVQWKVDNALQSGNSQESVTE QDSKDSTYSLSSTLTLSKADYEKHKVYACEVTHQGLSSPVTK SFNRGEC |

In a particular embodiment the antibody or antigen binding fragment thereof includes a heterologous constant domain. For example, the antibody includes a constant domain that is different from a native constant domain, such as a constant domain including one or more modifications to increase half-life.

In another particular embodiment, the antibody or antigen-binding fragment thereof according to the first aspect is: (a) multispecific; and/or (b) a recombinant antibody.

In another particular embodiment, the antibody or antigen-binding fragment thereof is an isolated antibody.

The term "recombinant" antibodies or antigen-binding fragments thereof, refers to such molecules created, expressed, isolated or obtained by technologies or methods known in the art as recombinant DNA technology which include, e.g., DNA splicing and transgenic expression. The term includes antibodies expressed in a non-human mammal (including transgenic non-human mammals, e.g., transgenic mice), or a cell (e.g., CHO cells) expression system, or a non-human cell expression system, or isolated from a recombinant combinatorial human antibody library. In some embodiments, a recombinant antibody shares a sequence with an antibody isolated from an organism (e.g., a mouse or a human), but has been expressed via recombinant DNA technology. Such antibodies may have post-translational modifications (e.g., glycosylation) that differ from the antibody as isolated from the organism.

"Multispecific" (e.g., bispecific or biparatopic) refers to molecules which include at least one first antigen-binding domain that specifically binds to SARS-CoV-2-S and at least one second antigen-binding domain that binds to a different antigen or to an epitope in SARS-CoV-2-S which is different from that of the first antigen-binding domain.

In another particular embodiment, the antibody is a monoclonal antibody. More particularly the monoclonal antibody is further processed to be a chimeric antibody, a humanized antibody, or a multispecific (e.g., bispecific antibody). In particular the antibody is a human monoclonal antibody. In a more particular antibody is a human IgG1 monoclonal antibody.

In another particular embodiment the antibody is an affinity matured antibody (produced by affinity matured B cells).

In another particular embodiment the antibody is a polyclonal or monoclonal humanized antibody.

In another particular embodiment of the first aspect, the antibody or antigen-binding fragment thereof specifically binds to a coronavirus SARS-CoV-2 spike protein. In particular it binds to the Receptor binding domain (RBD) of the SARS-CoV-2 spike protein. Particularly, it binds to a region located around residues 474-494 of the coronavirus SARS-CoV-2 spike protein according to the reference WH1 variant. In particular, the antibody or antigen-binding fragment thereof specifically binds to residues between 471 and 491 in the part of the spike protein of the Omicron BA.1 variant according to SEQ ID NO: 14.
SEQ ID NO:14 is

MFVFLVLLPLVSSQCVNLTTRTQLPPAYTNSFTRGVYYPDKVFRSSVLHSTQDLFLPFFSNVTWFHVISGTNG
TKRFDNPVLPFNDGVYFASIEKSNIIRGWIFGTTLDSKTQSLLIVNNATNVVIKVCEFQFCNDPFLDHKNNKSW
MESEFRVYSSANNCTFEYVSQPFLMDLEGKQGNFKNLREFVFKNIDGYFKIYSKHTPIIVREPEDLPQGFSALE
PLVDLPIGINITRFQTLLALHRSYLTPGDSSSGWTAGAAAYYVGYLQPRTFLLKYNENGTITDAVDCALDPLSET
KCTLKSFTVEKGIYQTSNFRVQPTESIVRFPNITNLCPFDEVFNATRFASVYAWNRKRISNCVADYSVLYNLAP
FFTFKCYGVSPTKLNDLCFTNVYADSFVIRGDEVRQIAPGQTGNIADYNYKLPDDFTGCVIAWNSNKLDSKVS
GNYNYLYRLFRKSNLKPFERDISTEIYQAGNKPCNGVAGFNCYFPLRSYSFRPTYGVGHQPYRVVVLSFELLH
APATVCGPKKSTNLVKNKCVNFNFNGLKGTGVLTESNKKFLPFQQFGRDIADTTDAVRDPQTLEILDITPCSFG
GVSVITPGTNTSNQVAVLYQGVNCTEVPVAIHADQLTPTWRVYSTGSNVFQTRAGCLIGAEYVNNSYECDIPI
GAGICASYQTQTKSHRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNFSQILPDPSKPSKRS
FIEDLLFNKVTLADAGFIKQYGDCLGDIAARDLICAQKFKGLTVLPPLLTDEMIAQYTSALLAGTITSGWTFGAGA
ALQIPFAMQMAYRFNGIGVTQNVLYENQKLIANQFNSAIGKIQDSLSSTASALGKLQDVVNHNAQALNTLVKQL
SSKFGAISSVLNDIFSRLDKVEAEVQIDRLITGRLQSLQTYVTQQLIRAAEIRASANLAATKMSECVLGQSKRVD
FCGKGYHLMSFPQSAPHGVVFLHVTYVPAQEKNFTTAPAICHDGKAHFPREGVFVSNGTHWFVTQRNFYEP
QIITTDNTFVSGNCDVVIGIVNNTVYDPLQPELDSFKEELDKYFKNHTSPDVDLGDISGINASVVNIQKEIDRLNE
VAKNLNESLIDLQELGKYEQYIKWPWYIWLGFIAGLIAIVMVTIMLCCMTSCCSCLKGCCSCGSCCKFDEDDSE
PVLKGVKLHYT.

In another embodiment, the antibody or antigen-binding fragment thereof according to the first aspect has one or more of the following characteristics:
(a) binds to SARS-CoV-2-S with binding affinity, expressed as K_{D} (Dissociation Constant), of at least about 10⁻⁹ M, or of at least about 10⁻¹⁰ M, or of at least about 10⁻¹¹ M, as measured by real-time surface plasmon resonance, e.g., BIACORE^{™};
(b) neutralizes SARS-CoV-2 WT and variants: WH1, D614G, Alpha, Beta, Gamma, Delta, Mu and Omicron variants BA.1, BA.2, BA.4, BA.5 in pseudo-neutralization assays, with an IC₅₀ below 200 ng/ml, or below 100 ng/ml, or below 50 ng/ml, or below 40 ng/ml, or below 20 ng/ml, or below 12 ng/ml;
(c) demonstrates an increase in survival and no changes in body weight in a SARS-CoV-2-infected animal after administration to said SARS-CoV-2-infected animal, as compared to a comparable SARS-CoV-2-infected animal without said administration;
(d) binds to the surface of SARS-CoV-2;
(e) protects mice engineered to express the human ACE2 or TMPRSS2 protein from death and/or weight loss caused by SARS-CoV-2 infection; and
(f) protects K18-hAC2 mice from SARS-CoV-2 infection.

The antibodies and antigen-binding fragments thereof disclosed in the present application can be obtained by synthetic routes, for example by solid-phase peptide synthesis on a rink amide resin using Fmoc-α-amine-protected amino acid. They can also be obtained from the recombinant gene technology, through genetic engineering of a fusion gene. This typically involves removing the stop codon from a cDNA sequence coding for the first protein, then appending the cDNA sequence of the second protein in frame through ligation or overlap extension PCR. That DNA sequence will then be expressed by a cell as a single protein. The protein can be engineered to include the full sequence of both original proteins, or only a portion or fragment of either. If the two entities are proteins, often linker (or "spacer") peptides are also added, which make it more likely that the proteins fold independently and behave as expected. Translation of this fusion gene results in a single or multiple polypeptides with functional properties derived from each of the original proteins.

As indicated above, the second aspect of the invention provides polynucleotides encoding any of the antibodies or antigen-binding fragments as defined in the first aspect. The invention additionally provides polynucleotides encoding any of the antibodies or antigen-binding fragments thereof as defined in the above embodiments.

A "polynucleotide" is to be understood as a nucleic acid molecule (DNA or RNA) comprising deoxyribonucleotides or ribonucleotides. Nucleic acid can be single or double stranded, and it includes, but it is not limited to, nucleotide sequences coding for polypeptides. When the polynucleotides are fusion genes, they are also known as "gene constructs". A "gene construct" according to the invention can also be named as an "expression cassette". It refers to a polynucleotide sequence including in turn a sequence coding for a protein of interest, which is operatively linked to an expression promoter, said promoter controlling expression of the sequence coding for the protein. For "operatively linked" is to be understood that the sequence coding for the protein is disposed after the sequence of the promoter (in the 5'-3' direction), or near the promoter in case restriction sites are included, or other stabilizing elements of the gene construction are present. The gene constructs (expression cassette) may also comprise small fragments with useful sequences to adapt it to more complex expression systems (vectors, plasmids), or a polyadenylation tail disposed after the sequence coding for the protein of interest. The expression cassette itself is also an expression system, being vectors or plasmids further used to protect the gene construct, or to promote entrance to cells in case of viral vectors. The "promoters" are of DNA regions that initiate transcription of particular genes. Promoters are located near the transcription start sites of genes, on the same strand and upstream on the DNA (towards the 5' region of the sense strand). Promoters are about 100-1000 base pairs long. A "constitutive promoter" is a promoter that is active in all circumstances in the cell, contrary to others that are regulated, becoming active in the cell only in response to specific stimuli, such as "inducible promoters". For the efficient expression of the polypeptide codified by the polynucleotide, the latter is inserted in a vector, which is a DNA molecule used as a vehicle to artificially carry foreign genetic material into another cell, where it can be replicated and/or expressed (e.g., plasmid, cosmid, Lambda phages). A vector containing foreign DNA is termed recombinant DNA. The four major types of vectors are plasmids, viral vectors, cosmids, and artificial chromosomes. These vectors may carry elements (sequences) the skilled person will know aiding in the expression and stability of the expressed sequence, as well as tags or labels to purify the recombinant expressed polypeptide. The vectors are transfected to appropriate host cells, which by means of their replicative and expression biochemical machinery will allow the obtaining of the recombinant polypeptide.

A skilled person would be aware that recombinant production of the above antibodies may proceed by expression of the light and heavy chains from two different expression vectors, one comprising the polynucleotide sequence for the light chain and the other comprising the polynucleotide sequence for the heavy chain. Alternatively, the antibody can be produced recombinantly by expressing the light and heavy chains from a single expression vector. Thus, the present application contemplates polynucleotides encoding for the light or heavy chain of the antibody defined in the first aspect, as well as polynucleotides encoding for both light and heavy chains of the antibody defined in the first aspect.

In the third aspect, the invention also provides expression vectors comprising the polynucleotides as disclosed above. Again, it is noted that the light and heavy chains of the antibody of the invention can be encoded in one expression vector or in two different expression vectors. Examples of suitable vectors include those conventionally used in biomedicine and known to the skilled person. In a particular embodiment, suitable vectors are the Abvec2.0-IGHG1, the Abvec1.1-IGKC.

And it also discloses in the fourth aspect a host cell which comprises the polynucleotide(s) and/or the vector(s) of the invention.

It also provides a cell culture comprising said host cell. The skilled person would know which host cells are suitable for the synthesis of the protein of the invention.

In a particular embodiment, the host cell is a mammalian cell line. In a more particular embodiment the host cell is a Human Embryonic Kidney cell line (HEK). And in a more particular embodiment the host cell is a Expi 293F mammalian cell line (suspension-adapted Human Embryonic Kidney (HEK)).

A fifth aspect of the invention refers to a method for preparing the antibodies as defined in the first aspect. The method comprising the steps of:
(a) introducing into a host cell one or more expression vectors as defined in the third aspect;
(b) culturing the host cell under conditions favorable for the expression of the one or more polynucleotides; and
(c) optionally, isolating the antibody or antigen-binding fragment from the host cell and/or a medium in which the host cell is grown.

In one embodiment one expression vector comprising the polynucleotides encoding for the light chain of the antibody or antigen-binding fragment as defined above and one expression vector comprising the polynucleotides encoding for the heavy chain of the antibody or antigen-binding fragment as defined above are introduced in the host cell. In another embodiment, one expression vector comprising both the polynucleotides encoding for the heavy chain and of the light chain of the antibody or antigen-binding fragment as defined above are introduced in the host cell.

In particular embodiments the expression vector(s) and the host cell are as defined above.

The sixth aspect relates to a pharmaceutical composition comprising a therapeutically effective amount of the antibody or antigen binding fragment as defined in the first aspect, and/or the polynucleotide as defined in the second aspect, and/or an expression vector as defined in the third aspect, and a pharmaceutically acceptable excipient and/or carrier.

The term "carrier" is to be understood as a pharmaceutically acceptable component other than the immunogenic component. The carrier can be organic, inorganic, or both. Suitable carriers are well known to those of skill in the art and include, without limitation, large, slowly metabolized macromolecules such as proteins, polysaccharides, polylactic acids, polyglycolic acids, polymeric amino acids, amino acid copolymers, lipid aggregates (such as oil droplets or liposomes) and inactive virus particles.

When applicable, the expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

In a particular embodiment, the pharmaceutical composition comprises a further therapeutic agent. In a more particular embodiment the said further therapeutic agent is selected from the group consisting of: a further antibody, or an antigen-binding fragment thereof, that binds a SARS-CoV-2 spike protein, an anti-inflammatory agent, for example an anti-inflammatory antibody such as sarilumab, tocilizumab, gimsilumab, tixagevimab, cilgavimab, PF-07321332, ritonavir, regdanvimab, casirivimab, imdevimab, remdesivir, and sotrovimab, an antimalarial agent such as chloroquine or hydroxychloroquine, an antibody or antigen-binding fragment thereof that binds TMPRSS2, an anti-viral drug and a vaccine.

The skilled person will understand that standard methods for the preparation of pharmaceutical compositions are adequate for the preparation of the pharmaceutical compositions comprising the antibodies of the invention. In the same way, the selection of the therapeutically effective amount of the antibodies or antigen-binding fragments, and of the accompanying excipients depending on the final form of administration (oral, parenteral, etc.) is that commonly used according to the skilled person in the art.

As defined in the seventh aspect, the antibody or antigen binding fragment as defined above, and/or the polynucleotide as defined in the second aspect, and/or the expression vectors as defined in the third aspect, and/or the pharmaceutical composition comprising any of the above, are for use in therapy.

The seventh aspect can also be formulated as the use of the antibody or antigen binding fragment thereof, and/or a nucleotide, and/or the expression vector, and/or the pharmaceutical composition as defined above for the manufacture of a medicament. This aspect can also be formulated as a method for the prevention and/or treatment of a disease, in an animal including humans, the method comprising administering an effective amount (i.e. therapeutically effective amount) of the antibody or antigen binding fragment thereof, and/or a nucleotide, and/or the expression vector, and/or the pharmaceutical composition as defined above in a subject in need thereof.

In a particular embodiment of the seventh aspect, the antibody or antigen binding fragment as defined in the first aspect, and/or the polynucleotide as defined in the second aspect, and/or the expression vectors as defined in the third aspect, and/or the pharmaceutical composition comprising any of the above, together with pharmaceutically acceptable excipients or carrier, are for use in the prevention and/or treatment of a disease caused by SARS-CoV-2. Or, in other words, for use in the prevention and/or treatment of COVID-19. More in particular, they are for use in the prevention and/or treatment of a disease caused by a SARS-CoV-2 infection. More in particular, the SARS-CoV-2 infection can be produced by a variant selected from the group: WH1, D614G, Alpha, Beta, Iota, Lambda, Gamma, Delta, Mu, and Omicron. More in particular, the Omicron variant is selected from the variants BA.1, BA.2, BA.4 and BA.5.

This embodiment can also be formulated as the use of the antibody or antigen binding fragment thereof as defined in the first aspect, and/or the polynucleotide as defined in the second aspect, and/or the expression vector as defined in the third aspect, and/or the pharmaceutical composition as defined in the fifth aspect for the manufacture of a medicament for the treatment and/or prevention of a disease caused by a SARS-CoV-2 infection in an animal including humans. More in particular, the SARS-CoV-2 infection can be produced by a variant selected from the group: WH1, D614G, Alpha, Beta, Iota, Lambda, Gamma, Delta, Mu and Omicron. More in particular, Omicron variant is selected from the variants BA.1, BA.2, BA.4 and BA.5. And it can also be formulated as a method for the prevention and/or treatment of a disease caused by a SARS-CoV-2 infection or COVID-19, in an animal including human, the method comprising administering an effective amount (i.e., therapeutically effective amount) of the antibody or antigen binding fragment thereof as defined in the first aspect, and/or the polynucleotide as defined in the second aspect, and/or the expression vector as defined in the third aspect, and/or the pharmaceutical composition as defined in the fifth aspect, together with pharmaceutically acceptable excipients or carriers, in a subject in need thereof, including a human. More in particular, the SARS-CoV-2 infection or COVID-19 can be produced by a variant selected from the group: WH1, D614G, Alpha, Beta, Iota, Lambda, Gamma, Delta, Mu and Omicron. More in particular, the Omicron variant is selected from the variants BA.1, BA.2, BA.4 and BA.5.

In a particular embodiment of the seventh aspect, the antibody or antigen binding fragment thereof as defined in the first aspect, and/or the polynucleotide as defined in the second aspect, and/or the expression vector as defined in the third aspect, and/or the pharmaceutical composition as defined in the fifth aspect, together with pharmaceutically acceptable excipients or carrier, are for use in combination with a further therapeutic agent. In a more particular embodiment, the further therapeutic agent is selected from the group consisting of: a further antibody, or an antigen-binding fragment thereof, that binds a SARS-CoV-2 spike protein, an anti-inflammatory agent, for example an anti-inflammatory antibody such as sarilumab, tocilizumab, gimsilumab, tixagevimab, cilgavimab, PF-07321332, ritonavir, regdanvimab, casirivimab, imdevimab, remdesivir, sotrovimab, an antibody or antigen-binding fragment thereof that binds TMPRSS2, or an anti-viral drug and a vaccine.

In a more particular embodiment, the antibody or antigen-binding fragment, or the expression vectors, or the pharmaceutical composition and the further therapeutic agent in the combined treatment are administered sequentially, simultaneously or within a therapeutic interval.

Herein disclosed is also a kit of parts that comprises:
(a) the antibody or antigen-binding fragment thereof as defined in the first aspect, and/or the polynucleotide as defined in the second aspect, and/or the expression vector as defined in the third aspect, and/or the pharmaceutical composition as defined in the fifth aspect, together with pharmaceutically acceptable excipients or carrier; and
(b) optionally a further therapeutic agent; and
(c) optionally, instructions for its use.

Herein disclosed is also a vessel or injection device which comprises the antibody or antigen-binding fragment thereof as defined in the first aspect, and/or the polynucleotide as defined in the second aspect, and/or the expression vector as defined in the third aspect, and/or the pharmaceutical composition as defined in the fifth aspect, together with pharmaceutically acceptable excipients or carrier.

In a particular embodiment, the kit, vessel or injection device as disclosed above are for use in therapy. In a particular embodiment, they are for use in the prevention and/or treatment of a disease caused by SARS-CoV-2. More particularly they are for use in the prevention and/or treatment of COVID-19. More in particular, they are for use in the prevention and/or treatment of a disease caused by a SARS-CoV-2 infection produced by the ancestral WH1 or another variant selected from the group: D614G, Alpha, Beta, Iota, Lambda, Gamma, Delta, Mu and Omicron. More in particular, Omicron is selected from the variants BA.1, BA.2, BA.4 and BA.5.

The eighth aspect of the invention relates to the use of the antibody or antigen-binding fragment as defined in the first aspect for the detection of SARS-CoV-2 in an isolated biological sample from a subject. In other words, the eighth aspect can also be formulated as the use of the antibody or antigen-binding fragment as defined in the first aspect as a tool or means for the diagnosis and/or prognosis of a disease caused by a SARS-CoV-2 infection in an isolated biological sample from a subject. In a particular embodiment the disease is COVID-19.

The eighth aspect can also be formulated as an in vitro method for the diagnosis and/or prognosis of a disease caused by a SARS-CoV-2 infection, which comprises determining the presence of SARS-CoV-2-S in an isolated sample from a subject, as an indicative of SARS-CoV-2 infection. In a particular embodiment, the method further comprises the steps of:
- incubating an isolated sample of a subject with the antibody and or antigen-binding fragment thereof as defined in the first aspect; and
- determining the presence of antibody-SARS-CoV-2-S complexes;
wherein the presence of antibody-SARS-CoV-2-S complexes is indicative of SARS-CoV-2 infection.

In a particular embodiment of all the different alternatives of the eighth aspect, the sample obtained from the subject is a tissue or a bodily fluid. In a particular embodiment the sample is selected from blood, plasma, serum, nasal scrap, oral tissue, oral scraping, oral wash, saliva, sweat or urine. And in a more particular embodiment, the sample is a nasal scrap, blood or saliva.

Exemplary assays for SARS-CoV-2-S may include, e.g., contacting a sample with an anti-SARS-CoV-2-S antibody or antigen-binding fragment thereof of the invention, wherein the anti-SARS-CoV-2-S antibody or antigen-binding fragment thereof is labeled with a detectable label or reporter molecule or used as a capture ligand to selectively isolate SARS-CoV-2-S from samples. The presence of an anti-SARS-CoV-2-S antibody or antigen binding fragment thereof complexed with SARS-CoV-2-S indicates the presence of SARS-CoV-2-S in the sample. Alternatively, an unlabeled anti-SARS-CoV-2-S antibody can be used in combination with a secondary antibody which is itself detectably labeled. The detectable label or reporter molecule can be a radioisotope, such as 3 H, 14 C, 32 P, 35 S, or 125 I; a fluorescent or chemiluminescent moiety such as fluorescein isothiocyanate, or rhodamine; or an enzyme such as alkaline phosphatase, β-galactosidase, horseradish peroxidase, or luciferase. Specific exemplary assays that can be used to detect or measure SARS-CoV-2-S in a sample include neutralization assays, enzyme-linked immunosorbent assay (ELISA), radioimmunoassay (RIA), and fluorescence-activated cell sorting (FACS). Thus, the present invention includes a method for detecting the presence of spike protein polypeptide in a sample comprising contacting the sample with an anti-SARS-CoV-2-S antibody or antigen-binding fragment thereof and detecting the presence of a SARS-CoV-2-S/anti-SARS-CoV-2-S antibody or antigen-binding fragment thereof wherein the presence of the complex indicates the presence of SARS-CoV-2-S.

An anti-SARS-CoV-2-S antibody or antigen-binding fragment thereof of the invention may be used in a Western blot or immune-protein blot procedure for detecting the presence of SARS-CoV-2-S or a fragment thereof in a sample. Such a procedure forms part of the present invention and includes the steps of e.g.: (1) providing a membrane or other solid substrate comprising a sample to be tested for the presence of SARS-CoV-2-S, e.g ., optionally including the step of transferring proteins from a sample to be tested for the presence of SARS-CoV-2-S (e.g., from a PAGE or SDS-PAGE electrophoretic separation of the proteins in the sample) onto a membrane or other solid substrate using a method known in the art (e.g ., semi-dry blotting or tank blotting); and contacting the membrane or other solid substrate to be tested for the presence of SARS-CoV-2-S or a fragment thereof with an anti-SARS-CoV-2-S antibody or antigen-binding fragment thereof of the invention.

Such a membrane may take the form, for example, of a nitrocellulose or vinyl-based (e.g., polyvinylidene fluoride (PVDF)) membrane to which the proteins to be tested for the presence of SARS-CoV-2-S in a non-denaturing PAGE (polyacrylamide gel electrophoresis) gel or SDS-PAGE (sodium dodecyl sulfate polyacrylamide gel electrophoresis) gel have been transferred (e.g., following electrophoretic separation in the gel). Before contacting the membrane with the anti-SARS-CoV-2-S antibody or antigen-binding fragment thereof, the membrane is optionally blocked, e.g., with non-fat dry milk or the like so as to bind non-specific protein binding sites on the membrane.
(2) washing the membrane one or more times to remove unbound anti-SARS-CoV-2-S antibody or antigen binding fragment thereof and other unbound substances; and
(3) detecting the bound anti-SARS-CoV-2-S antibody or antigen-binding fragment thereof.

Detection of the bound antibody or antigen-binding fragment thereof indicates that the SARS-CoV-2-S protein is present on the membrane or substrate and in the sample. Detection of the bound antibody or antigen-binding fragment thereof may be by binding the antibody or antigen-binding fragment thereof with a secondary antibody (an anti-immunoglobulin antibody) which is detectably labeled and, then, detecting the presence of the secondary antibody label.

The anti-SARS-CoV-2-S antibodies and antigen-binding fragments disclosed herein may also be used for immunohistochemistry. Such a method forms part of the present invention and comprises, e.g.,
(1) contacting tissue to be tested for the presence of SARS-CoV-2-S protein with an anti-SARS-CoV-2-S antibody or antigen-binding fragment thereof of the invention; and
(2) detecting the antibody or antigen-binding fragment thereof on or in the tissue.

If the antibody or antigen-binding fragment thereof itself is detectably labeled, it can be detected directly. Alternatively, the antibody or antigen binding fragment thereof may be bound by a detectably labeled secondary antibody wherein the label is then detected.

In the present invention it is also disclosed a complex comprising an antibody or antigen-binding fragment thereof as defined in the first aspect bound to a SARS-CoV-2 spike protein polypeptide; to an antibody or antigen-binding fragment thereof that competes with the antibody or antigen-binding fragment thereof as defined in the first aspect for binding to SARS-CoV-2 spike protein polypeptide; and to an antibody or antigen-binding fragment thereof that binds to the same epitope as, or to an overlapping epitope on, SARS-CoV-2 spike protein polypeptide.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1: mAbs generation and neutralization assays

### Human subjects and sample collection

Blood samples were collected from COVID-19 convalescent individuals infected with SARS-CoV-2 during the first wave of the COVID-19 pandemic in Spain. Diagnosis of SARS-CoV-2 infection was confirmed by reverse transcription-quantitative polymerase chain reaction (RT-qPCR) of nasopharyngeal swab. Peripheral blood mononuclear cells (PBMCs) were isolated from whole blood collected with EDTA anticoagulant via Ficoll-Paque Premium (Cytiva, Freiburg, Germany) following the manufacturer's instructions. PBMCs were resuspended in fetal bovine serum (FBS; Gibco, Riverside, MA, USA) with 10% dimethyl sulphoxide (DMSO; Sigma-Aldrich, St. Louis, USA) and stored in liquid nitrogen prior to use. All procedures were approved by the Ethical Committee for Clinical Investigation of the Institut Hospital del Mar d'Investigacions Mèdiques (Number 2020/9189/I).

### Single B-cell FACS sorting

For the isolation of WH1 SARS-CoV-2 spike-specific B cells, 26.4 pmol His-Tagged Biotinylated SARS-CoV-2 (2019-nCoV) Spike RBD Recombinant Protein (Sino Biological Inc.; cat number: 40592-VO8H-B) was firstly incubated for 1 hours with 3.78 pmol Streptavidin Alexa Fluor 647 (Thermo Fisher) and Streptavidin Alexa Fluor 488 (Thermo Fisher), separately. Next, PBMCs were incubated with the fluorescently labelled RBD probes and with a cocktail of fluorescent conjugated antibodies containing anti-CD19 Pe-Cy7, anti-IgM BV605, anti HLA-DR AF700, anti CD38 APC-cy7, anti Igλ light chain PerCP-Cy5.5 (all from Biolegend), anti-IgD PE CF594 (BD Bioscience), anti-CD21 PE-cy5 (BD Bioscience) and anti-IgA Viogreen (Miltenyi). Dead cells were excluded using 4'-6'-diamidine-2'-phenylindole (DAPI) (Sigma). Alive DAPI⁻CD19⁺RBD⁺ B cells were single-cell index sorted using a FACSAria II (BD Biosciences) into empty 96-well PCR plates (VWR). FACSDiva software (Becton Dickinson) was used for acquisition and FlowJo for post-sorting analysis. Plates were immediately sealed with foil, frozen on dry ice and stored at -80 °C.

### Expression-cloning of antibodies

Antibodies were identified and sequenced as previously described (Wardemann H, Busse CE. Expression Cloning of Antibodies from Single Human B Cells. Methods Mol Biol. 1956:105-125, 2019. DOI: 10.1007/978-1-4939-9151-8_5. PMID: 30779032). In brief, RNA from single cells was reverse transcribed in the original 96-well sort plate using random hexamers (Thermo Fisher), 0.76% NP 40 detergent solution (Thermo Fisher), RNasin ribonuclease inhibitor (Promega), DTT (Invitrogen) and Superscript III Reverse Transcriptase (Invitrogen, 180080-044).The resulting cDNA was stored at -20 °C for subsequent amplification of the variable IGH, IGL and IGK genes by nested PCR and sequencing using reverse and forward primers listed in

(Wardemann H, Busse CE. Expression Cloning of Antibodies from Single Human B Cells. Methods Mol Biol.; 1956:105-125, 2019. DOI: 10.1007/978-1-4939-9151-8_5. PMID: 30779032) and in the Table B. Sequences were analyzed using Change-O (IgBlast). Following analysis, Ig gene-specific PCR was performed for the successfully annotated transcripts. Amplicon from the first PCR reaction were used as templates for cloning into antibody expression vectors (Abvec2.0-IGHG1 for the heavy chain and Abvec1.1- IGKC or Abvec1.1.-IGLC2-Xhol for of the light chains, all from AddGene). Recombinant antibodies were produced by transient co-transfection of Expi293 cells using Expi293 expression System (Thermo Fisher). Recombinant human IgG1 monoclonal antibodies were purified on HiTrap MabSelect Sure Columns (Merck) from cell supernatant 5 days after transfection.

**Table B. Primers used to amplify the variable domains of the heavy and light immunoglobulin chains of the mAb17T2 (Wardemann H, Busse CE. Expression Cloning of Antibodies from Single Human B Cells. Methods Mol Biol. 1956:105-125, 2019. DOI: 10.1007/978-1-4939-9151-8_5. PMID: 30779032). Wherein "R" represents "A" or "G".**

| PCR step | Primer name | Primer sequence (5'→ 3') | SEQ ID; |
|---|---|---|---|
| IGH first PCR | Forward | | |
| | hIGHV-1/7-066-fw | ACAGGTGCCCACTCCCAGG TGCAG | SEQ ID NO: 15 |
| | hIGHV-1/7-017-fw | ATGGACTGGACCTGGAG | SEQ ID NO: 16 |
| | hIGHV-1/7-041-fw | TCCTCTTTGTGGTGGCAGCA GC | SEQ ID NO: 17 |
| | hIGHV-2-035-fw | TCCACGCTCCTGCTRCTGAC | SEQ ID NO: 18 |
| | hIGHV-3-066-fw | AAGGTGTCCAGTGTGARGTG CAG | SEQ ID NO: 19 |
| | hIGHV-3-057-fw | TAAAAGGTGTCCAGTGT | SEQ ID NO: 20 |
| | hIGHV-4/6-066-fw | CCCAGATGGGTCCTGTCCCA GGTGCAG | SEQ ID NO: 21 |
| | hIGHV-4-022-fw | ATGAAACACCTGTGGTTCTT CC | SEQ ID NO: 22 |
| | hIGHV-5-066-fw | CAAGGAGTCTGTTCCGAGGT GCAG | SEQ ID NO: 23 |
| | Reverse | | |
| | hIGHM-082-rv | GGAAGGAAGTCCTGTGCGA GGC | SEQ ID NO: 24 |
| | hIGHA-111-rv | GTCCGCTTTCGCTCCAGGTC ACACT | SEQ ID NO: 25 |
| | hIGHG-137-rv | GGAAGGTGTGCACGCCGCT GGTC | SEQ ID NO: 26 |
| IGL first PCR | Forward | | |
| | hIGL V-1-068-fw | GGTCCTGGGCCCAGTCTGT GCTG | SEQ ID NO: 27 |
| | hIGLV-2-068-fw | GGTCCTGGGCCCAGTCTGC CCTG | SEQ ID NO: 28 |
| | hIGLV-3-068-fw | GCTCTGTGACCTCCTATGAG CTG | SEQ ID NO: 29 |
| | hIGLV-4/5-068-fw | GGTCTCTCTCSCAGCYTGTG CTG | SEQ ID NO: 30 |
| | hIGLV-6-068-fw | GTTCTTGGGCCAATTTTATG CTG | SEQ ID NO: 31 |
| | hIGLV-7-068-fw | GGTCCAATTCYCAGGCTGTG GTG | SEQ ID NO: 32 |
| | hIGLV-8-083-fw | GAGTGGATTCTCAGACTGTG GTG | SEQ ID NO: 33 |
| | Reverse | | |
| | hIGLC-057-rv | CACCAGTGTGGCCTTGTTGG CTTG | SEQ ID NO: 34 |
| IGK first PCR | Forward | | |
| | hIGKV-1-060-fw | ATGAGGSTCCCYGCTCAGCT GCTGG | SEQ ID NO: 35 |
| | hIGKV-3-049-fw | CTCTTCCTCCTGCTACTCTG GCTCCCAG | SEQ ID NO: 36 |
| | hIGKV-4-049-fw | ATTTCTCTGTTGCTCTGGAT CTCTG | SEQ ID NO: 37 |
| | Reverse | | |
| | hIGKC-172-rv | GTTTCTCGTAGTCTGCTTTG CTCA | SEQ ID NO: 38 |
| IGH second PCR | Forward | | |
| | hIGHV-pan-080-fw | AGGTGCAGCTGCTGGAGTC KGG | SEQ ID NO: 39 |
| | Reverse | | |
| | hIGHA-076-rv | GGAAGAAGCCCTGGACCAG GC | SEQ ID NO: 40 |
| | hIGHG-074-rv | AGTCCTTGACCAGGCAGCC C | SEQ ID NO: 41 |
| | hIGHM-031-rv | GGGAATTCTCACAGGAGAC GA | SEQ ID NO: 42 |
| IGL second PCR | Forward | | |
| | hIGLV-pa1-fw | CAGYCTGYSCTGACTCA | SEQ ID NO: 43 |
| | hIGLV-pa2-fw | TCCTATGAGCTGACWCAG | SEQ ID NO: 44 |
| | Reverse | | |
| | hIGLC-026-rv | TCAGAGGAGGGYGGGAACA GAGTG | SEQ ID NO: 45 |
| IGK second PCR | Forward | | |
| | hIGKV-pan-fw | ATGACCCAGWCTCCABYCW CCCTG | SEQ ID NO: 46 |
| | Reverse | | |
| | hIGKC-032-rv | AACTGCTCATCAGATGGCGG G | SEQ ID NO: 47 |
| IGH gene-specific PCR | Forward | | |
| | 5' Agel VH1 | CTGCAACCGGTGTACATTCC CAGGTGCAGCTGGTGCAG | SEQ ID NO: 48 |
| | Reverse | | |
| | 3'Sall JH3 | TGCGAAGTCGACGCTGAAG AGACGGTGACCATTG | SEQ ID NO: 49 |
| IGK gene-specific PCR | Forward | | |
| | 5'Agel V_{K}3-20 | CTGCAACCGGTGTACATTCA GAAATTGTGTTGACGCAGTC T | SEQ ID NO: 50 |
| | Reverse | | |
| | 3' BsiWI Jk1/2/4 | GCCACCGTACGTTTGATYTC CACCTTGGTC | SEQ ID NO: 51 |
| Insert check PCR | Ab sense | GCTTCGTTAGAACGCGGCTA C | SEQ ID NO: 52 |
| | IgG internal | GTTCGGGGAAGTAGTCCTTG AC | SEQ ID NO: 53 |

### Production of Recombinant SARS-CoV-2 Proteins

RBD sequences from Beta (β), and Gamma (γ) variants were obtained from the World Health Organization tracking of variants (https://www.who.int/en/activities/tracking-SARS-CoV-2-variants/) and Pango lineage classification (https://cov-lineages.org/). DNA fragments encoding the RBD from β (first identified in South Africa, B.1.351: K417N, E484K, N501Y) and γ (first identified in Japan/Brazil, P.1: K417T, E484K, N501Y) variants were synthetized by integrated DNA technology (IDT) as gblocks and codon optimized for mammalian expression. The fragments were inserted in a pCAGGS vector using Gibson Assembly. RBD proteins were expressed in-house in Expi293F human cells (Thermo Fisher Scientific) by transfection of the cells with purified DNA and polyethylenimine (PEI). RBD from the original SARS-CoV-2 (Wuhan or WH1 variant) and from Delta (δ) (first identified in India, B.1.617.2: L452R, T478K), Omicron BA.1 and Omicron BA.2, variants were purchased from Sino Biological.

### ELISAs

96-well half-area flat bottom high-bind microplates (Corning) were coated overnight at 4°C with the different SARS-CoV-2 RBD recombinant viral proteins at 2 µg/ml in PBS (30 µl per well). Plates were washed with PBS 0.05% Tween 20 (PBS-T) and blocked with blocking buffer (PBS containing 1.5% Bovine serum albumin, BSA) for 2 hours at RT. Monoclonal antibodies were serially diluted (starting dilution at 10 µg/mL and followed by 11 serial dilutions of ratio 1:4) in PBS supplemented with 0.05% Tween 20 and 1% BSA and added to the viral protein- or PBS-coated plates for 2 hours at RT. After washing, plates were incubated with horseradish peroxidase (HRP)-conjugated anti-human IgG secondary antibody (Southern Biotech, 2042-05) diluted 1:4000 in PBS containing 0.05% Tween 20 and 1% BSA for 45 min at RT. Plates were washed 5 times with PBS-T and developed with TMB substrate reagent set (BD bioscience) with development reaction stopped with 1 M H₂SO₄. Absorbance was measured at 450 nm on a microplate reader (Infinite 200 PRO, Tecan). Optical density (OD) measurement was obtained after subtracting the absorbance at 570 nm from the absorbance at 450 nm. The EC₅₀ was determined using four-parameter nonlinear regression (GraphPad Prism).

### Cell lines

HEK293T cells overexpressing wild-type (WT) human ACE-2 (Integral Molecular, USA) were used as target in the pseudovirus-based neutralization assay. Cells were maintained in T75 flasks with Dulbecco's Modified Eagle's Medium (DMEM) supplemented with 10% FBS and 1 µg/ml puromycin (Thermo Fisher Scientific, USA). Expi293F cells (Thermo Fisher Scientific) are a HEK293 cell derivative adapted for suspension culture that were used for SARS-CoV-2 pseudovirus production. Cells were maintained under continuous shaking in

Erlenmeyer flasks following manufacturer's guidelines.

### Pseudovirus generation and neutralization assay

HIV reporter pseudoviruses expressing SARS-CoV-2 Spike protein and Luciferase were generated as previously described (Pradenas E, et al., Virological and Clinical Determinants of the Magnitude of Humoral Responses to SARS-CoV-2 in Mild-Symptomatic Individuals. Front. Immunol. 13:860215, 2022. doi: 10.3389/fimmu.2022.860215). pNL4-3.Luc.R-E- vector was obtained from the NIH AIDS Reagent Program (Ruth I. Connor, et al., Vpr Is Required for Efficient Replication of Human Immunodeficiency Virus Type-1 in Mononuclear Phagocytes, Virology, Volume 206, Issue 2, 935-944, 1995. doi: https://doi.org/10.1006/viro.1995.1016. Available at https://www.sciencedirect.com/science/article/pii/S0042682285710161). SARS-CoV-2.SctΔ19 was generated (GeneArt) from the full protein sequence of SARS-CoV-2 spike by deletion of the last 19 amino acids of the C-terminal (26), was human-codon optimized and inserted into pcDNA3.1 (+). A similar procedure was followed to generate expression plasmids for the D614G, alpha, beta, delta, mu, omicron BA.1, omicron BA.2, omicron BA.4/BA.5 variants of SARS-CoV-2 S protein according to consensus data (www.outbreak.info). Of note is that BA.4 and BA.5 share the spike protein and differ on other regions of their genome, that is why the binding affinity of the antibody is the same. Expi293F cells were transfected using ExpiFectamine293 Reagent (Thermo Fisher Scientific) with pNL4-3.Luc.R-.E- and SARS-CoV-2.SctΔ19 at an 8:1 ratio, respectively. Control pseudoviruses were obtained by replacing the S protein expression plasmid with a VSV-G protein expression plasmid as reported (Trinité, B., et al., SARS-CoV-2 infection elicits a rapid neutralizing antibody response that correlates with disease severity. Sci Rep 11, 2608 (2021). https://doi.org/10.1038/s41598-021-81862-9). Supernatants were harvested 48 hours after transfection, filtered at 0.45 µm, frozen, and titrated on HEK293T cells overexpressing WT human ACE-2 (Integral Molecular, USA). This neutralization assay has been previously validated in a large subset of samples and negative controls with a replicative viral inhibition assay (Trinité, B., et al., SARS-CoV-2 infection elicits a rapid neutralizing antibody response that correlates with disease severity. Sci Rep 11, 2608 (2021). https://doi.org/10.1038/s41598-021-81862-9). Neutralization assays were performed in duplicate. Briefly, in Nunc 96-well cell culture plates (Thermo Fisher Scientific), 200 TCID50 of pseudovirus were preincubated with three-fold serial dilutions of purified antibodies for 1 hour at 37°C. Then, 2 × 10⁴ HEK293T/hACE2 cells treated with DEAE-Dextran (Sigma-Aldrich) were added. Results were read after 48 hours using the EnSight Multimode Plate Reader and BriteLite Plus Luciferase reagent (PerkinElmer, USA). The values were normalized, and the ID₅₀ (reciprocal dilution inhibiting 50% of the infection) and ID₈₀ were calculated by plotting and fitting all duplicate neutralization values and the log of plasma dilution to a 4-parameters equation in Prism 9.0.2 (GraphPad Software, USA).

### Results

### Reactivity and Neutralizing capacity of recombinant mAb17T2 against SARS-CoV-2 and variants

Several mAbs were generated as human monoclonal IgG1 and screened in vitro to confirm the reactivity profile by ELISA and the neutralization activity against multiple variants by pseudo-neutralization assays. Among the identified mAbs, mAb17T2 was the only one able to bind the receptor binding domain (RBD) of SARS-CoV-2 spike proteins of all variants tested, including the WH1 (ancestral variant) and the subsequent Beta (B.1.351), Gamma (P.1), Delta (B.1.617.2), Omicron BA.1 and Omicron BA.2 with EC₅₀ values below 10 ng/ml (Table 1).

| | **mAb 17T2** |
|---|---|
| | **EC₅₀ (µg/ml)** |
| **WH1** | 0.004700023 |
| **Beta (B.1.351)** | 0.003758374 |
| **Gamma (P.1)** | 0.003076805 |
| **Delta (B.1.617.2)** | 0.003384543 |
| **Omicron (BA.1)** | 0.007121968 |
| **Omicron (BA.2)** | 0.008107743 |

Table 1. EC₅₀ values for binding of mAb17T2 to the RBD of SARS-CoV-2 ancestral variant and variants Beta, Gamma, Delta, Omicron BA.1 and Omicron BA.2.

mAb17T2 also showed an exceptionally high neutralization activity in pseudotyped virus neutralization assays with SARS-CoV-2 spike proteins from the following variants: WH1 (ancestral variant), D614G (the first mutation fixed in the genome of the new virus) and the subsequent alpha, Beta, Gamma, Delta, Mu and Omicron (BA.1). Comparing to other mAbs, mAb17T2 retained an unaltered potency against Omicron (BA.1) with an IC₅₀ (half-maximal Inhibitory Concentration) lower than 3 ng/ml (Table 2).

To note, mAb17T2 was developed as a human IgG1 recombinant monoclonal antibody but was generated starting from an RBD-specific IgA+ canonical memory B cell (RBD⁺CD19⁺IgM⁻IgD⁻IgA⁺CD27⁺CD21⁺HLA-DR⁺CD11c⁻ λ light chain-).

**Table 2. Lentiviruses pseudotyped with SARS-CoV-2 spike proteins from WH1, D614G, Alpha, Beta, Gamma, Delta, Mu, Omicron (BA.1) were incubated with serial dilutions of the human monoclonal antibody mAb17T2 and IC₅₀, IC₈₀ and IC₉₀ were determined.**

| | **mAb17T2** | | |
|---|---|---|---|
| | IC₅₀ (µg/ml) | IC₈₀ (µg/ml) | IC₉₀ (µg/ml) |
| **WH1** | 0.04061 | 0.080433182 | 0.119905409 |
| **D614G** | 0.007925 | 0.015340228 | 0.022574477 |
| **Alpha (B.1.1.7)** | 0.006656 | 0.014001254 | 0.02163752 |
| **Beta (B.1.351)** | 0.002198 | 0.004807893 | 0.007600036 |
| **Gamma (P.1)** | 0.01257 | 0.021830105 | 0.030163344 |
| **Delta (B.1.617.2)** | 0.01048 | 0.016509003 | 0.021546669 |
| **Mu (B.1.621)** | 0.009065 | 0.014039273 | 0.018142164 |
| **Omicron (BA.1)** | 0.002646 | 0.005201347 | 0.007719296 |

### Comparison of the neutralization activity of mAb17T2 against multiple variants (including Omicron BA.1, BA.2, BA.4 and BA.5) with two commercial monoclonal antibodies known to neutralize Omicron

To further confirm the broadly neutralizing activity of mAb17T2, it was compared with that of two well described broadly SARS-CoV-2 neutralizing human monoclonal antibodies: S309 and S2E12.

S309 was first isolated from memory B cells from a recovered SARS-CoV-1 patient (Pinto, D., et al. Cross-neutralization of SARS-CoV-2 by a human monoclonal SARS-CoV antibody. Nature 583, 290-295 (2020). https://doi.org/10.1038/s41586-020-2349-y). S309 targets the Spike protein of the virus, which modulates the viral entry into host cells. S309 targets a specific residue on the Spike protein, N343, which was later determined to be a consistently conserved glycan in the Sarbecovirus subgenus, including SARS-CoV-2. An engineered form of the parental monoclonal S309, known as Sotrovimab, has received emergency use authorization from the US food and drug administration.

S2E12 is an ultrapotent human monoclonal IgG1 antibody isolated from an individual recovering from severe COVID-19 (Tortorici et al., Ultrapotent human antibodies protect against SARS-CoV-2 challenge via multiple mechanisms. Science 370,6519. 2020. DOI: 10.1126/science.abe3354), that has been recently reported to maintain potent neutralization activity against SARS-CoV-2 variants including Omicron (Zhou et al., Structural basis for potent antibody neutralization of SARS-CoV-2 variants including B.1.1.529. Science Vol 376, Issue6581. 2022. DOI: 10.1126/science.abn8897).

In this second set of experiments, additional pseudoviruses were generated and tested, namely the pseudoviruses exposing the new omicron subvariant BA.2 and BA4/5 spike glycoproteins.

Results summarized in Table 3 confirm the results obtained in the previous experiment. They also show that mAab17T2 has higher neutralizing activity than S309 antibody, but lower potency than S2E12 antibody for most of pre-Omicron variants tested. However, compared to S2E12, mAb17T2 showed 12.2- and 13.5-fold neutralizing activity against Omicron subvariants BA1 and BA2, respectively. Moreover, compared to S309, mAb17T2 showed 46- and 41-fold neutralizing activity against Omicron subvariants BA1 and BA2, respectively. Only mAb17T2 is highly efficient in neutralizing the new BA.4 and BA.5 Omicron variants.

**Table 3. Lentiviruses pseudotyped with SARS-CoV-2 spike proteins from WH1, D614G, Alpha, Beta, Gamma, Delta, Mu and Omicron (BA.1, BA.2 and BA.4/5) were incubated with serial dilutions of the human monoclonal antibodies mAb17T2, S309 and S2E12 and IC₅₀ was determined.**

| | **IC₅₀ (µg/ml)** | | | | | | | | |
|---|---|---|---|---|---|---|---|---|---|
| | **SARS-COV-2** | | | | | | | | |
| **mAb** | **WH1** | **D614G** | **Alpha** | **Beta** | **Gamma** | **Delta** | **BA.1** | **BA.2** | **BA.4/5** |
| **17T2** | 0.0383 | 0.01 | 0.0069 | 0.0027 | 0.0083 | 0.0096 | 0.0032 | 0.0101 | 0.00203 |
| **S309** | 0.2744 | 0.0802 | 0.0347 | 0.0043 | 0.0083 | 0.0336 | 0.148 | 0.4165 | 0.1422 |
| **S2E12** | 0.0005 | 0.0004 | 0.0004 | <0.0003 | 0.0003 | <0.0003 | 0.0393 | 0.1372 | >1.0 |

### Example 2: prophylactic antibody treatment in mice

### SARS-CoV-2 infection and prophylactic antibody treatment in K18-hACE2 mice

All animal procedures were performed under the approval of the Committee on the Ethics of Animal Experimentation of the IGTP and the authorization of Generalitat de Catalunya (code: 11222). B6.Cg-Tg(K18-ACE2)2Prlmn/J (or K18-hACE2) hemizygous transgenic mice (034860, Jackson Immunoresearch, West Grove, PA, United States) were bred, genotyped and maintained at CMCiB as stated in Tarrés-Freixas et al.,2022 (Tarrés-Freixas et al., Heterogeneous Infectivity and Pathogenesis of SARS-CoV-2 Variants Beta, Delta and Omicron in Transgenic K18-hACE2 and Wildtype Mice. Frontiers in Microbiology, 13, 2022. Doi: 10.3389/fmicb.2022.840757). A total of 24 adult K18-hACE2 hemizygous mice (aged 6-14 weeks) were used in this experiment, distributed in sex-balanced groups. One day before SARS-CoV-2 challenge, mice were anesthetized with isoflurane (FDG9623; Baxter, Deerfield, IL, USA) and administered by intraperitoneal injection with either 10 mg/kg mAb17T2 (n = 10) or isotype control (IgGb12) (n = 10).

After 24 hours, treated animals were challenged with 1000 TCID50 of Omicron BA.1.1 SARS-CoV-2 isolate (EPI_ISL_8151031), or PBS (uninfected control group). Viral challenge was performed under isoflurane anesthesia in 50 µl PBS (25 µl/nostril). All mice fully recovered from the infection and anesthesia procedures. Body weight and clinical signs were monitored daily from the antibody injection until the end of the experiment.

Five animals per treated group and two per uninfected group were euthanized at 3- and 7-days post-infection (dpi) for viral RNA quantification and pathological analyses. No animals had to be sacrificed due to Humane Endpoint Criteria considering body weight loss and clinical signs (Tarrés-Freixas et al., Heterogeneous Infectivity and Pathogenesis of SARS-CoV-2 Variants Beta, Delta and Omicron in Transgenic K18-hACE2 and Wildtype Mice. Frontiers in Microbiology, 13, 2022. Doi: 10.3389/fmicb.2022.840757). Euthanasia was performed under deep isoflurane anesthesia by whole blood extraction via intracardiac puncture and confirmed by cervical dislocation. Serum was separated from whole blood by centrifugation at 4000 g for 10 min. Oropharyngeal swab, lung, brain, and nasal turbinate were collected for cell free viral RNA quantification. Extracted tissues were fixed by immersion in 10% buffered formalin for histological and immunohistochemistry analysis. SARS-CoV-2 PCR detection and viral load quantification of the samples was performed as described in Tarrés-Freixas et al.,2022 (Tarrés-Freixas et al., Heterogeneous Infectivity and Pathogenesis of SARS-CoV-2 Variants Beta, Delta and Omicron in Transgenic K18-hACE2 and Wildtype Mice. Frontiers in Microbiology, 13, 2022. Doi: 10.3389/fmicb.2022.840757)

### Histopathology and SARS-CoV-2 immunohistochemistry

Nasal turbinate, lung and brain from mice were collected on days 3 and 7 post-SARS-CoV-2 inoculation, were fixed by immersion in 10% buffered formalin and embedded into paraffin blocks. The histopathological analysis was performed on slides stained with hematoxylin/eosin and examined by optical microscopy. A semi-quantitative score based on the level of inflammation (0-No lesion; 1-Mild, 2-Moderate or 3-Severe lesion) was established based on previous classifications (Brustolin et al., Protection against reinfection with D614- or G614-SARS-CoV-2 isolates in golden Syrian hamster, Emerging Microbes & Infections, 10:1, 797-809, 2021. DOI: 10.1080/22221751.2021.1913974; Vidal et al., Chronological brain lesions after SARS-CoV-2 infection in hACE2-transgenic mice. Vet Pathol. 2022 Jul;59(4):613-626. DOI: 10.1177/03009858211066841).

SARS-CoV-2 nucleoprotein was detected by immunohistochemistry (IHC) using the rabbit monoclonal antibody (40143-R019, Sino Biological) at a 1:15000 dilution. For immunolabelling visualization, the EnVision^{®}+ System linked to horseradish peroxidase (HRP, Agilent-Dako) and 3,3'-diaminobenzidine (DAB) were used. The amount of viral antigen in tissues was semi-quantitatively scored (0-No antigen detection, 1-Low, 2-Moderate and 3-High amount of antigen) following previously published classifications (Brustolin et al., Protection against reinfection with D614- or G614-SARS-CoV-2 isolates in golden Syrian hamster, Emerging Microbes & Infections, 10:1, 797-809, 2021. DOI: 10.1080/22221751.2021.1913974; Vidal et al., Chronological brain lesions after SARS-CoV-2 infection in hACE2-transgenic mice. Vet Pathol. Jul;59(4):613-626. 2022. DOI: 10.1177/03009858211066841).

### Results

Compared to the isotype control (IgGb12), a single prophylaxis dose of mAb17T2 conferred significant reduction of viral burden (copies/ml) in the lungs at day 7 post-infection (Isotype control, mean: 410911723; mAb17T2, mean: 199446; p=0.045) (Table 4), nasal turbinates at day 7 post-infection (Isotype control, mean: 5004628; mAb17T2, mean: 100217; p=0.009) (Table 4) and swab (Isotype control, mean: 31781; mAb17T2, mean: 3423; p=0.009) in the K18-hACE2 mice at 7 days after infection. Significant reduction in viral burden was also observed in the lung at day 3 post-infection (Isotype control, mean: 4109258683; mAb17T2, mean: 1284525; p=0.009).

**Table 4. Omicron BA.1.1 SARS-CoV-2 viral load (copies/ml) in the lung or nasal turbinate of K18-hACE2 mice treated with IgGb12 isotype control or mAb17T2 antibody.**

| LUNG | | | |
|---|---|---|---|
| Day 3 | | Day 7 | |
| Viral load (copies/ml) | | Viral load (copies/ml) | |
| Isotype Ct | mAb17T2 | Isotype Ct | mAb17T2 |
| 1366072972 | 5070048 | 472412500 | 378146 |
| 1347342222 | 1082566 | 925614694 | 1000 |
| 14304349333 | 1000 | 48035 | 1000 |
| 1339070333 | 24775 | 99451205 | 567757 |
| 2189458556 | 244238 | 557032181 | 49327 |
| | | | |

| NASAL TURBINATE | | | |
|---|---|---|---|
| Day 3 | | Day 7 | |
| Viral load (copies/ml) | | Viral load (copies/ml) | |
| Isotype Ct | mAb17T2 | Isotype Ct | mAb17T2 |
| 313266049 | 8307240 | 15432513 | 257611 |
| 114968583 | 392743 | 6582434 | 1564 |
| 7845951 | 133298 | 1975081 | 13769 |
| 615308 | 15433805 | 579799 | 220951 |
| 2652436 | 713235 | 453313 | 7191 |

Significant differences were also observed with respect of histopathological lesions and SARS-CoV-2 focal expression in lung tissues at day 3 (Histology, p=0.00157; no observed lesions in mice treated with mAb17T2; IHC, p values 0.0135 only one mouse treated with mAb17T2 showed minimal detection of virus replication) and day 7 post-infection (histology, p=0.0578; and IHC, p=0.00982). (Table 5)

**Table 5. Histology (0=no viral antigen, 1=low amount of viral antigen, multifocal) and IHC (0=no lesion, 1=mild lesion, 2=moderate lesion) score in lungs of Omicron BA.1.1 SARS-CoV-2 infected K18-hACE2 mice treated with IgGb12 isotype control or mAb17T2 antibody.**

| LUNG (HISTOLOGY SCORE) | | | |
|---|---|---|---|
| Day 3 | | Day 7 | |
| Isotype Ct | mAb17T2 | Isotype Ct | mAb17T2 |
| 0 | 1 | 0 | 1 |
| 0 | 1 | 0 | 1 |
| 0 | 1 | 0 | 0 |
| 0 | 1 | 0 | 1 |
| 0 | 1 | 1 | 1 |
| | | | |

| LUNG (IHC SCORE) | | | |
|---|---|---|---|
| Day 3 | | Day 7 | |
| Isotype Ct | mAb17T2 | Isotype Ct | mAb17T2 |
| 0 | 1 | 0 | 1 |
| 1 | 1 | 0 | 1 |
| 0 | 2 | 0 | 0 |
| 0 | 1 | 0 | 1 |
| 0 | 1 | 0 | 1 |

### Example 3: Cryo-Electron Microscopy

3D reconstruction from Cryo-Electron Microscopy (cryo-EM) is used to determine the mode of recognition and molecular interaction that are required for the antibodies to function and bind to target SARS-CoV-2 spike protein. Fab protein was generated from mAb17T2 by enzyme digestion. Fab fragment was incubated with SARS-CoV-2 B.1.1.529 (Omicron) S1+S2 trimer Protein (Sino Biological). The complexed material was then analyzed by cryogenic electron microscopy. 3D reconstruction analysis combined with in silico prediction using AlphaFold artificial intelligence program indicates that MAb17T2 binds to the spike protein with RBD domains in the up position. This analysis also reveals that mAb17T2 recognizes specific residues in the receptor-binding motif (RBM) of the spike protein. Binding surface of the antibody/RBD interaction in the trimeric Spike glycoprotein of Omicron BA.1 variant involved residues 471-491 of the Omicron BA.1 Spike protein sequence as set forth in SEQ ID NO: 14. This binding surface of the antibody/RBD interaction corresponds to the positions 474-494 of the reference WH1 variant spike protein sequence as set forth in SEQ ID NO: 13.

### Citation List

### Non Patent Literature

- Tongqing Zhou et al., "Structural basis for potent antibody neutralization of SARS-CoV-2 variants including B.1.1.529", Science 2022, vol. 376, issue 6591, https://doi.org/10.1126/science.abn8897
- Bruel, T. et al. "Serum neutralization of SARS-CoV-2 Omicron sublineages BA.1,BA.2, BA.4 and BA.5 in patients receiving monoclonal antibodies". Nat Med 2022, https://doi.org/10.1038/s41591-022-01792-5
- Needleman and Wunsch, J. Mol. Biol. 48: 443-453, 1970. DOI: 10.1016/0022-2836(70)90057-4
- EMBOSS: The European Molecular Biology Open Software Suite, Rice et al., , Trends Genet. 16: 276-277, 2000. DOI: 10.1016/s0168-9525(00)02024-2
- Altschul et al., "Basic local alignment search tool", 1990, J. Mol. Biol, v. 215, pages 403-410, 1990. https://doi.org/10.1016/S0022-2836(05)80360-2
- Wardemann H, Busse CE. Expression Cloning of Antibodies from Single Human B Cells. Methods Mol Biol. 1956:105-125, 2019. DOI: 10.1007/978-1-4939-9151-8_5. PMID: 30779032
- Pradenas E, et al., Virological and Clinical Determinants of the Magnitude of Humoral Responses to SARS-CoV-2 in Mild-Symptomatic Individuals. Front. Immunol. 13:860215, 2022. doi: 10.3389/fimmu.2022.860215
- Ruth I. Connor, et al., Vpr Is Required for Efficient Replication of Human Immunodeficiency Virus Type-1 in Mononuclear Phagocytes, Virology, Volume 206, Issue 2, 935-944, 1995. doi:
   https://doi.org/10.1006/viro.1995.1016. Available at
   https://www.sciencedirect.com/science/article/pii/S0042682285710161
- Trinité, B., et al., SARS-CoV-2 infection elicits a rapid neutralizing antibody response that correlates with disease severity. Sci Rep 11, 2608 (2021). https://doi.org/10.1038/s41598-021-81862-9
- Pinto, D., et al. Cross-neutralization of SARS-CoV-2 by a human monoclonal SARS-CoV antibody. Nature 583, 290-295 (2020). https://doi.org/10.1038/s41586-020-2349-y
- Tortorici et al., Ultrapotent human antibodies protect against SARS-CoV-2 challenge via multiple mechanisms. Science 370,6519. 2020. DOI: 10.1126/science.abe3354
- Tarrés-Freixas et al., Heterogeneous Infectivity and Pathogenesis of SARS-CoV-2 Variants Beta, Delta and Omicron in Transgenic K18-hACE2 and Wildtype Mice. Frontiers in Microbiology, 13, 2022. Doi: 10.3389/fmicb.2022.840757
- Brustolin et al., Protection against reinfection with D614- or G614-SARS-CoV-2 isolates in golden Syrian hamster, Emerging Microbes & Infections, 10:1, 797-809, 2021. DOI: 10.1080/22221751.2021.1913974
- Vidal et al., Chronological brain lesions after SARS-CoV-2 infection in hACE2-transgenic mice. Vet Pathol. Jul;59(4):613-626, 2022. DOI: 10.1177/03009858211066841

## Claims

1. An antibody, or antigen-binding fragment thereof, comprising a light chain comprising a light chain variable region (LCVR) that comprises three light chain complementarity determining regions (LCDRs) set forth in SEQ ID NO: 1 (LCDR1), SEQ ID NO: 2 (LCDR2), and SEQ ID NO: 3 (LCDR3); and a heavy chain comprising a heavy chain variable region (HCVR) that comprises three heavy chain complementarity determining regions (HCDRs) set forth in SEQ ID NO: 4 (HCDR1), SEQ ID NO: 5 (HCDR2), and SEQ ID NO: 6 (HCDR3).

2. The antibody or antigen-binding fragment thereof according to claim 1, wherein the LCVR comprises a sequence set forth in SEQ ID NO: 7, or a sequence at least 80% identical to SEQ ID NO: 7; and/or wherein the HCVR comprises a sequence set forth in SEQ ID NO: 8, or a sequence at least 80% identical to SEQ ID NO: 8.

3. The antibody or antigen-binding fragment thereof according to any one of claims 1-2, wherein the heavy chain further comprises an immunoglobulin constant region, or an IgG1 constant region, or a human IgG1 constant region; and the light chain further comprises an immunoglobulin constant region, or an IgK constant region, or a human IgK constant region.

4. The antibody or antigen-binding fragment thereof according to any one of claims 1-3, wherein:
- the heavy chain comprises a sequence set forth in SEQ ID NO: 11, or a sequence at least 80% identical to SEQ ID NO:11; and
- the light chain comprises a sequence set forth in SEQ ID NO: 12, or a sequence at least 80% identical to SEQ ID NO:12.

5. The antibody or antigen-binding fragment thereof according to any one of claims 1-4, wherein the antibody neutralizes SARS-CoV-2 in pseudo-neutralization assays, with an IC₅₀ below 100 ng/ml.

6. The antibody or antigen-binding fragment thereof according to any one of claims 1-5, wherein the antibody is an isolated antibody.

7. A polynucleotide encoding an antibody or antigen-binding fragment thereof as defined in any one of claims 1-6.

8. An expression vector comprising a polynucleotide as defined in claim 7.

9. A host cell comprising the polynucleotide as defined in claim 7, and/or the expression vector as defined in claim 8.

10. A pharmaceutical composition comprising a therapeutically effective amount of the antibody or antigen binding fragment as defined in any one of claims 1-6, or the polynucleotide as defined in claim 7, or the expression vector as defined in claim 8, and a pharmaceutically acceptable excipient and/or carrier.

11. The antibody or antigen-binding fragment as defined in any one of claims 1-6, or the polynucleotide as defined in claim 7, or the expression vector as defined in claim 8, or the pharmaceutical composition as defined in claim 10, for use in therapy.

12. The antibody or antigen-binding fragment as defined in any one of claims 1-6, or the polynucleotide as defined in claim 7, or the expression vector as defined in claim 8, or the pharmaceutical composition as defined in claim 10, which is for use in the prevention and/or treatment of a disease caused by SARS-CoV-2.

13. The antibody or antigen binding-fragment as defined in any one of claims 1-6, or the polynucleotide as defined in claim 7, or the expression vector as defined in claim 8, or the pharmaceutical composition as defined in claim 10, for use according to claims 10-12, wherein the disease is a SARS-CoV-2 infection produced by a variant selected from the group: WH1, D614G, Alpha, Beta, Iota, Lambda, Gamma, Delta, Mu and Omicron.

14. The antibody or antigen-binding fragment as defined in any one of claims 1-6, or the polynucleotide as defined in claim 7, or the expression vector as defined in claim 8, or the pharmaceutical composition as defined in claim 10, for use according to any one of claims 10-13, which is for use in combination with a further therapeutic agent.

15. Use of the antibody or antigen-binding fragment as defined in any one of claims 1-6 for the detection of SARS-CoV-2 in an isolated biological sample.
